# EUROPEAN PATENT APPLICATION

(11) **EP 4 209 576 A1**
(43) Date of publication of application: **12.07.2023**
(21) Application number: 22151043.1
(22) Date of filing: 11.01.2022
(51) Int. Cl.: C12M 1/12, C12M 1/26, C12M 1/36, C12M 1/00

(54) **METHOD FOR OPERATING A CLARIFICATION SETUP**

(71) Applicant: Sartorius Stedim North America Inc., Bohemia, NY 11716 (US)
(72) Inventor: Soeldner, Robert, 37083 Göttingen (DE); Austerjost, Jonas, 33397 Rietberg (DE); Saballus, Martin, 37249 Neu-Eichenberg (DE)
(74) Representative: Gottschald Patentanwälte Partnerschaft mbB

(57) **Abstract**

The invention is directed to a method for operating a clarification setup (1) of a bioprocess installation (2), wherein the clarification setup (1) comprises a fluidized bed centrifuge (3) for the clarification of a cell broth by centrifugation, wherein the fluidized bed centrifuge (3) comprises at least two centrifuge chambers (9-12), wherein the fluidized bed centrifuge (3) is being operated in a forward operation for a particle loading cycle and/or a particle washing cycle and in a backward operation for a particle discharging cycle, wherein during the particle loading cycle, cell broth loaded into the centrifuge chamber (9-12) proceeds to form a growing particle accumulation in the centrifuge chamber (9-12), wherein the clarification setup (1) comprises a monitoring sensor arrangement (17) with at least one, preferably optical, sensor (18) for producing monitoring sensor data, which are being transmitted to the electronic process control (14). It is proposed that in a monitoring routine, for at least part of the centrifuge chambers (9-12), monitoring sensor data (19) are being produced individually by the monitoring sensor arrangement (17) and that during the loading cycle, in an adjusting routine, at least one parameter of the clarification setup (1) is being adjusted based on the monitoring sensor data (19) as to control the loading of the cell broth the centrifuge chambers (9-12) individually according to a predefined adjusting strategy.

## Description

The present invention relates to a method for operating a clarification setup of a bioprocess installation according to the general part of claim 1, to a clarification setup according to claim 13 and to an electronic process control according to claim 14.

The term "bioprocess" presently represents any kind of biotechnological process, in particular a biopharmaceutical process. An example of such a bioprocess is the use of a bioreactor to cultivate microorganisms or mammalian cells under given conditions, wherein a cell broth is transferred from the bioreactor to a downstream process. The term "bioreactor" in this context means any manufactured device or system that supports a biological environment by allowing monitoring and control of at least one parameter. The term "bioproduct" presently represents any kind of compound produced in such a bioprocess. Examples for such bioproducts are proteins, in particular antibodies, growth factors or hormones, metabolites or any other molecule as well as cells or cellular components, such as organelles.

The method in question for operating a clarification setup may be applied in various fields of biotechnology. High efficiency in this field has been driven by the increasing demand for bioproducts, such as biopharmaceutical drugs. The efficiency in this sense is regarding not only the cost-effectiveness of the components to be used but also the controllability of the processes connected thereto. The method in question relies on the optimal filling of centrifuge chambers to optimize controllability as well as time-effectiveness of the bioprocess.

A known method for operating a clarification setup (EP 2 310 486 B1) comprises a fluidized bed centrifuge with a number of centrifuge chambers, which are each assigned a chamber inlet and a chamber outlet. The clarification setup also comprises a pumping arrangement assigned to the fluidized bed centrifuge and a liquid network for the transport of the liquid, which is the cell broth to be clarified. Finally, the clarification setup comprises an electronic process control for controlling at least the fluidized bed centrifuge and the pumping arrangement.

The known clarification setup with more than one centrifuge chamber generally allows for high output performance, as the centrifugation processes for each centrifuge chamber run in parallel. However, running those various centrifugation processes in parallel is complex, when maximum efficiency is intended. This is because the centrifugation processes in the centrifuge chambers each are to be seen as separate processes each with a unique dynam ic.

Another known method ("Operation and Maintenance Manual kSep400", Sartorius Stedim Biotech), which is the starting point for the invention, relies on the production of monitoring sensor data from a camera sensor, which monitoring sensor data are being analyzed manually by a user. Based on the experience of this user, those monitoring sensor data are being utilized to operate the clarification setup. Although the use of a camera sensor facilitates the analysis by the user, maximizing the efficiency of the clarification setup remains a challenge due to the above noted reasons.

It is therefore an object of the present invention to improve the known method for operating a clarification setup such that the bioprocess efficiency is improved with as little effort as possible.

The above noted problem is solved by a method for operating a clarification setup according to the general part of claim 1 with the features of the characterizing part of claim 1.

The basic idea underlying the invention is to control the loading of the cell broth for at least part of the centrifuge chambers individually based on monitoring sensor data, which, for example, may represent the individual particle filling level of the respective centrifuge chamber.

The term "particle" is to be understood in a broad sense and means any particulate solid compound, in particular cells, cell debris, proteins, (magnetic) microbeads or the like, which may be of biological or non-biological origin.

The term "particle filling level" is to be understood in a broad sense. The particle filling level is a measure for the filling of centrifuge chambers with particles and can be reflected by a percentage particle filling of the centrifuge chamber, a specific particle mass, volume, concentration or the like. Moreover, the particle filling level can be represented by a specific particle filling level at a given time or as a change in particle filling level over time, thereby also being reflective of the particle filling rate.

The term "individual" here generally means that the respective task, here the control of the loading, is performed for each centrifuge chamber individually. In particular, synchronization of the loading of the centrifuge chambers may be performed in various ways according to a predefined adjusting strategy.

Depending on the adjusting strategy, it is for example possible to enable a uniformly filling of each centrifuge chamber. This allows for a simultaneous discharge of all centrifuge chambers, hence enabling a more efficient usage of the centrifuge chamber capacities with low effort.

The synchronized loading of the centrifuge chambers reduces unnecessary waiting times, minimizes the number of centrifugation cycles necessary to process the bioreactor volume and may prevent particle breakthrough of valuable cells. The term "particle breakthrough" relates to the point in time during the fluidized bed centrifugation process, when the at least one centrifuge chamber is filled to capacity with particles, preferably cells, and wherein a continued loading of the centrifuge chamber leads to an overfilling of said centrifuge chamber.

The proposed, individual control of the loading of the respective centrifuge chambers can also be advantageous in view of the aim of a mechanically balanced operation of the rotor of the fluidized bed centrifuge, which carries the centrifuge chambers. An unbalance occurs when the inertia axis of the rotor with respect to the centrifuge axis is offset the centrifuge axis. This may be caused, for example, by an uneven loading of the centrifuge chambers with particles e.g. cells during the loading cycle. By a specific synchronized loading and with it a predefined distribution of the weight of the particles over the centrifuge chambers, a balanced operation may be achieved or at least be supported.

In detail, it is proposed, that in a monitoring routine, for at least part of the centrifuge chambers, monitoring sensor data are being produced individually by the monitoring sensor arrangement and that during the loading cycle, in an adjusting routine, at least one parameter of the clarification setup is being adjusted by the electronic process control based on the monitoring sensor data as to control the loading of the cell broth for at least part of the centrifuge chambers individually according to a predefined adjusting strategy. Again, the term "individual" means that the respective centrifuge chambers are each treated individually, here in terms of producing monitoring sensor data and in terms of the loading of cell broth.

According to a preferred embodiment, it has been found, that due to the floating state of the particles within the centrifuge chamber, the particle accumulation is proceeding in an ordered and reproducible way. In a wide range, this effect is taking place independently from the media to be processed. In particular it has been found that this leads to a visible phase boundary, which, with ongoing particle accumulation, is travelling across the centrifuge chamber, wherein the location of the phase boundary represents the objective filling level of the centrifuge chamber. It is particularly interesting that it has been realized that, in contrast to known non-fluidized bed centrifuges, the proposed invention leads to a visible phase boundary without exposing the centrifuged cells to such high acceleration forces. Such high acceleration forces could otherwise lead to cell damage and even cell death. Hence, the cells are here separated under milder conditions, leading to a higher viability, which is crucial in case the cells are to be re-used in a subsequent bioprocess. Additionally, with the proposed invention, the cell number within a centrifuge chamber can be estimated more accurately. This is due to the fact that particle accumulation under non-fluidized bed conditions is fundamentally different, since the acceleration forces acting on the cells are much higher. This is why in the proposed solution the cells in the particle accumulation are not as densely packed as they would be under non-fluidized bed conditions. Thereby, the particle filling level can be calculated more precisely. Also, this is particularly advantageous for a more precise calculation of the required cell number that is necessary in order to inoculate a potential subsequent bioprocess. Moreover, it has been found, that the above noted, systematic particle accumulation allows for the systematic calculation of the filling level based on a calculation model, which may even be performed in real time.

According to claim 2, in detail, it is therefore proposed, that in the monitoring routine, image-related data representing an optical image of the centrifuge chamber content are being produced by the monitoring sensor arrangement, which is, further preferably, according to claim 3, the basis for the calculation of a particle filling level of the respective centrifuge chamber.

By using automatic filling level determination via image processing, an accurate signal for each chamber can be acquired. As an image-related approach, the determined filling level is independent from broth composition, such as cell viability, cell types, and cell diameter, and is even able to reflect the presence of impurities or dead cells.

The individual control of the loading of at least part of the centrifuge chambers is preferably realized by individually controlling at least part of the pumps of the pumping arrangement according to claim 4. Preferably, the pump rate of each of those pumps is then controlled according to the adjusting strategy. This approach of controlling the loading of cell broth is easy to realize in an engineering view.

Claims 5 to 10 are directed to preferred adjusting strategies, which are based on specific control of the individual particle filling level of the respective centrifuge chamber. This direct control of the individual particle filling level of the respective centrifuge chamber leads to a simple way to increase effectiveness, as proposed in claims 6 to 10. While claims 6 and 7 propose to reach a target, for example a target particle filling level, individually for each centrifuge chamber, claims 8 to 10 focus on controlling the particle filling levels for at least part of the centrifuge chambers relatively to each other.

A preferred measure to achieve the above noted, mechanical balancing of the rotor of the centrifuge is subject of claim 11, taking account a balancing model, which represents the interrelation between a difference between particle filling levels of at least two centrifuge chambers and an unbalance of the rotor. With this it is possible to prevent an unbalanced state of the rotor from the beginning.

As an alternative, claim 12 proposes to take into account the sensor signal of a vibration sensor for minimizing the amplitudes of those vibrations, which are caused by an unbalanced operation of the rotor of the centrifuge. This is done by way of controlling the volumetric flow rates of at least part of the pumps assigned to centrifuge chambers in view of the detected vibrations.

According to a second independent teaching according to claim 13, the clarification setup for performing the proposed method is claimed as such. All explanations given for the proposed method are fully applicable to the proposed clarification setup.

A third independent teaching according to claim 14 is directed to an electronic process control. The electronic process control is designed to perform the proposed method according to any one of the preceding claims. Again, all explanations given before are fully applicable to this proposed third teaching. The electronic process control preferably comprises a data processing system for the realization of the proposed method.

A fourth teaching, that may be claimed independently as well, is directed to a computer program product for the proposed electronic process control. The computer program product is configured to realize the proposed method, in particular, to realize the above-noted routines. Again, all explanations given for the proposed method are fully applicable to the proposed computer program product.

A fifth teaching, that may be claimed independently as well, is directed to a computer-readable storage media, on which the computer program is stored. Again, all explanations given for the proposed method are fully applicable to the proposed readable storage media.

In the following, an embodiment of the invention is explained with respect to the drawings. The drawings show in
- Fig. 1: schematically a preferred embodiment of a proposed bioprocess installation, with which a proposed method is executable,
- Fig. 2: perspectively the working principle of the proposed method according to Fig. 1,
- Fig. 3: the individual filling levels of the centrifuge chambers of the centrifuge shown in Fig. 1 during a loading cycle a) without applying the proposed adjusting routine, b) with applying the proposed adjusting routine and
- Fig. 4: the centrifuge and the pumping arrangement according to Fig. 1.

The proposed method for operating a clarification setup 1 of a bioprocess installation 2 is preferably assigned to the upstream and downstream processes of a bioprocess, processing a liquid, in particular a cell broth for cell cultivation and/or bioproduction.

The term "liquid" is to be understood in a broad sense. It includes not only a pure liquid as such, but also emulsions and suspensions, e.g. a heterogeneous mixture of at least two different liquids or a heterogeneous mixture of solid particles and liquid.

The term "cell broth" is a suspension of particles in a solvent, in particular cells and/or cell debris in media. It describes in particular the entirety of the cultivation medium and the respective organism cultured in the cultivation medium.

The term "upstream process" involves all the steps related to cell bank, inoculum (seed train) development, media development, optimization of growth kinetics and the cultivation process itself as well as the corresponding in-process control. The harvest of cells can be seen as both, part of upstream- and part of downstream processing. The term "downstream process" involves all the steps related to the recovery and the purification of bioproducts, particularly biopharmaceuticals, from natural sources such as animal or plant tissue or cell broth, including the recycling of salvageable components and the proper treatment and disposal of waste.

In general, the cultivation of cells is currently used for the production of biopharmaceuticals, in particular proteins, such as human insulin, growth factors, hormones, vaccines, or antibodies, antibody derivatives, or the like. The bioproducts may as well be non-biopharmaceuticals, such as enzymes for food processing, laundry detergent enzymes, biodegradable plastics or biofuels. The focus of the present invention is on biopharmaceutical products secreted by the cells into the supernatant, such as antibodies or exosomes. Additionally or alternatively, the product can be the cells themselves, in particular mammalian cells including stem cells or immune cells such as CAR-T cells for the treatment of cancer.

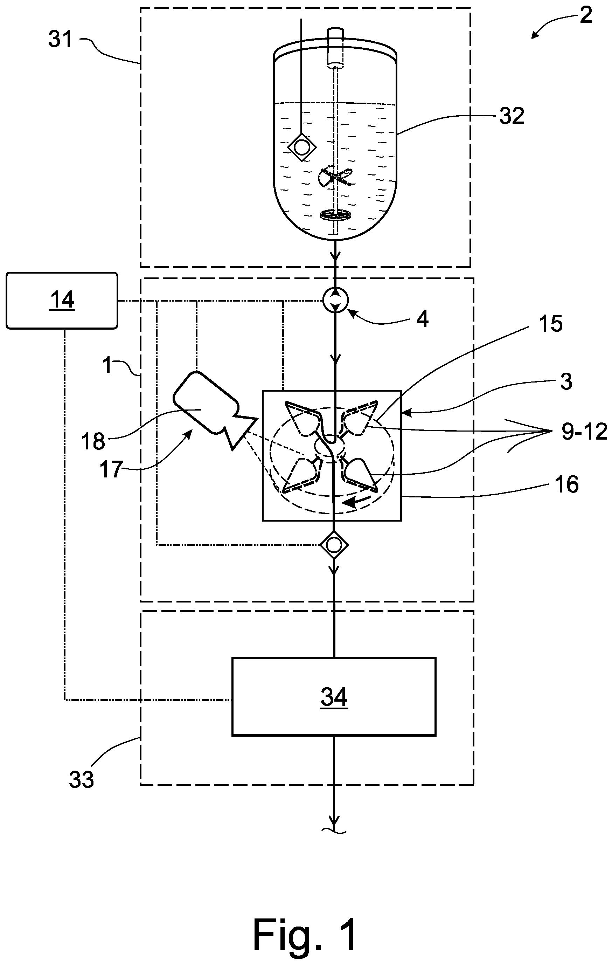

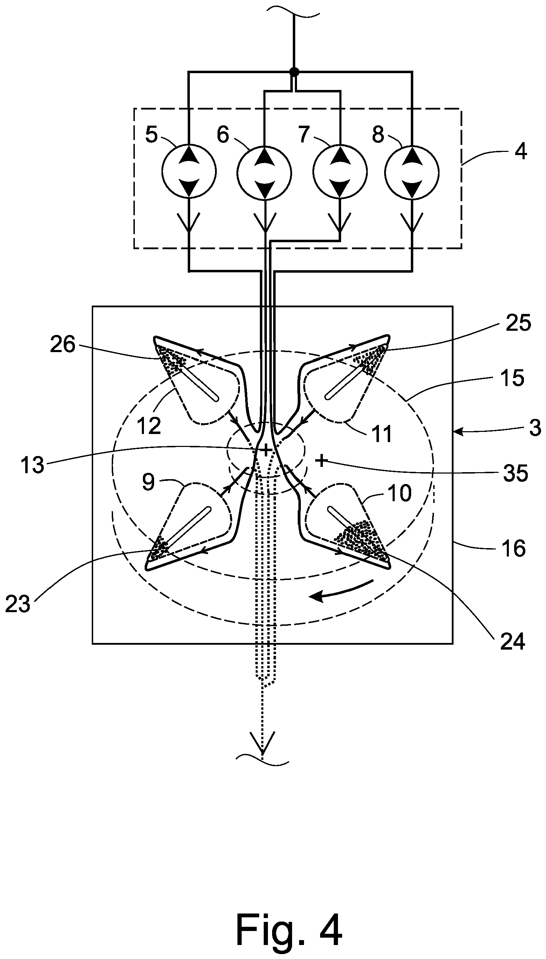

As shown in Fig. 1 to 4, according to all embodiments, the proposed method for operating a clarification setup 1 of a bioprocess installation 2 employs at least one fluidized bed centrifuge 3 for the clarification of a cell broth by centrifugation and a pumping arrangement 4 assigned to the fluidized bed centrifuge 3. The pumping arrangement 4 comprises at least two pumps 5-8, here and preferably four pumps 5-8. The fluidized bed centrifuge 3 comprises at least one centrifuge chamber 9-12, preferably an even number of centrifuge chambers 9-12, further preferably exactly four centrifuge chambers 9-12, which are being turned around a geometrical centrifuge axis 13 and develop a fluidized bed during normal operation. As shown in Fig. 4, preferably, each centrifuge chamber 9-12 comprises at least one assigned pump 5-8, here and preferably one single pump 5-8, for pumping liquid in or out of the chamber 9-12. The bioprocess installation 2 further comprises an electronic process control 14 for controlling at least the fluidized bed centrifuge 3 and the pumping arrangement 4.

"Centrifugation" is a term for sedimentation of particles in an artificially, by centrifugal forces created, gravitational field, wherein a significant reduction of separation time is achieved via large accelerating forces.

Here, the centrifuge is designed as a fluidized bed centrifuge 3 for performing a continuous centrifugation process. Preferred setups of the fluidized bed centrifuge 3 are described in EP 2 485 846 A1, the contents of which are hereby incorporated by reference herein.

The fluidized bed centrifuge 3 comprises a rotor 15 with the centrifuge chambers 9-12 attached thereto within the centrifuge housing 16. The rotor 15 may be rotated around the single geometrical centrifuge axis 13 by a, preferably electric, motor. The expression "single" means, that all chambers 9-12, which are carried by the rotor 15, rotate around one and the same geometrical centrifuge axis 13. The centrifuge revolution speed and the pumping rate are adjustable by the electronic process control 14, with the aim to establish a fluidized bed of particles, such as cells or cell debris, in the fluidized bed centrifuge 3. A fluidized bed is achieved when the centrifugal force on a particle is equal to the opposing fluid flow force so that a zero net force is exerted on the particle.

According to Fig. 1, the cell broth is being led through the fluidized bed centrifuge 3. The fluidized bed centrifuge 3 is being operated in a forward operation for a loading cycle and/or a washing cycle and in a backward operation for a particle discharging cycle.

"Forward operation" means one out of two possible fluid flow directions in a fluidized bed centrifuge and describes the operation leading to a separation of liquid and solid particles, such as media and cells. This "forward operation" allows, on the one hand, a washing of separated cells with buffer or media, preferably cultivation media, further preferably enriched media, and/or, on the other hand, the clarification of the cell broth. The goal here is to clarify the liquid supernatant from solid particles such as cells, cell debris, etc., which solid particles are considered biomass. The product to be obtained in this forward operation is the supernatant of the cell broth containing a bioproduct of interest, e.g., a recombinant protein, in particular an antibody.

The term "enriched media" describes media that comprise higher concentrations of vitamins, growth factors, trace nutrients, as well as carbon-source, nitrogen-source and/or amino acid concentrations or the like and, preferably, allow the respective organism to grow at its maximum growth rate due to the optimized nutrient concentrations. Growth factors and trace nutrients are included in the media for organisms incapable of producing all of the vitamins they require themselves. Inorganic nutrients, including trace elements such as iron, zinc, copper, manganese, molybdenum and cobalt are typically present in unrefined carbon and nitrogen sources but may have to be added when purified carbon and nitrogen sources are used.

The loading cycle refers to a cycle of loading the respective centrifuge chamber 9-12 in forward operation of the fluidized bed centrifuge 3 with cell broth to be centrifuged. Hence, during the particle loading cycle, cell broth loaded into the centrifuge chamber 9-12 proceeds to form a growing particle accumulation in the centrifuge chamber 9-12.

The washing cycle refers to a cycle of washing the respective centrifuge chamber 9-12 in forward operation of the fluidized bed centrifuge 3 with media or buffer. This washing cycle preferably serves for a supply of fresh nutrients to the cells.

Alternatively, the fluidized bed centrifuge 3 can be operated in a backward operation. "Backward operation" means the second out of two possible fluid flow directions in the fluidized bed centrifuge 3 and describes the operation leading to a discharge of the separated solid particles, preferably cells. The product to be obtained in backward operation are the cells in the cell broth.

Hence, the discharging cycle refers to the cycle assigned to the fluidized bed centrifuge 3, wherein in backward operation the centrifuge chamber 9-12 is drained from solid particles, preferably cells. This discharging cycle can i.a. serve for a re-use of cells in a subsequent bioprocess.

Moreover, the clarification setup 1 comprises a monitoring sensor arrangement 17 with at least one, here and preferable optical, sensor 18 for producing monitoring sensor data, which are being transmitted to the electronic process control 14. The optical sensor 18 is directed to the respective centrifuge chamber 9-12, as is shown in Fig. 1.

It is particularly essential for the invention, that in a monitoring routine, for at least part of the centrifuge chambers 9-12, monitoring sensor data 19 are being produced individually by the monitoring sensor arrangement 17. From these monitoring sensor data 19, preferably, the electronic process control 14 calculates a particle filling level 20 based on a calculation model, as will be explained later.

Further, it is essential for the invention, that during the loading cycle, in an adjusting routine, at least one parameter of the clarification setup 1 is being adjusted by the electronic process control 14 based on the monitoring sensor data 19 as to control the loading of the cell broth for at least part of the centrifuge chambers 9-12, here and preferably for all of the centrifuge chambers, individually according to a predefined adjusting strategy. This means that the monitoring sensor data, that have been produced for individual centrifuge chambers 9-12, is the basis for controlling the loading of the cell broth for those individual centrifuge chambers 9-12. With this, according to the predefined adjusting strategy, an individual control of the loading of each and any centrifuge chamber 9-12 is possible with almost unrestricted flexibility.

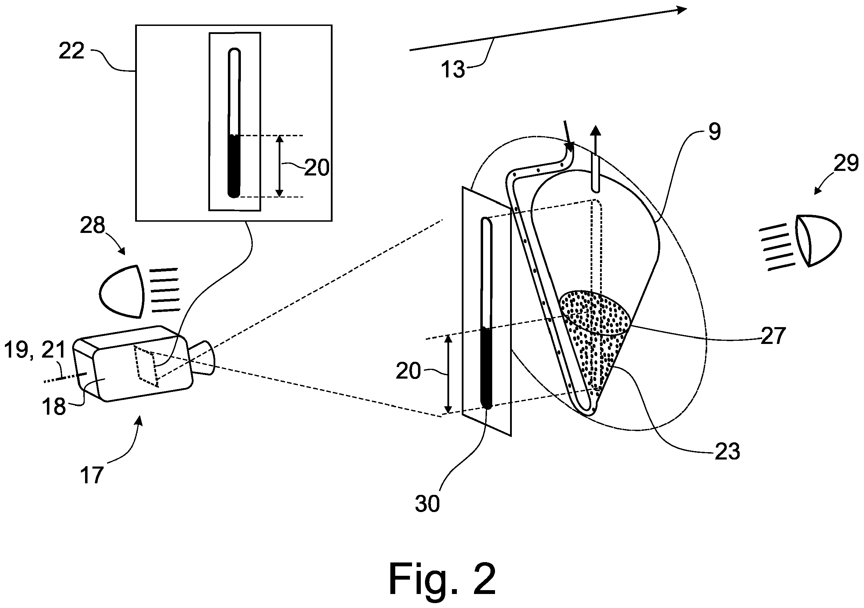

As shown in Fig. 2, in the monitoring routine, image-related data 21, which are representing an optical image of the respective centrifuge chamber content 23-26 are being produced as the monitoring sensor data 19 by the monitoring sensor arrangement 17.

Preferably, the monitoring sensor data 19 represent the particle filling level 20 of the respective centrifuge chambers 9-12. This particle filling level 20 may also be derived from the monitoring sensor data 19, preferably by calculation based on a calculation model.

The calculation model is to be understood as a rule system for the calculation of the filling level 20 based on the image related data 21. In a preferred embodiment, the calculation model may be exchanged between two different operating instances or even within one and the same operating instance. Moreover, the calculation model is highly adaptable to different bioprocess settings leading to altered optical properties, such as regarding the choice of the used cultivation media which can lead to different contrasts, different brightnesses, different densities or the like. According to another preferred embodiment, the calculation model can be adapted to different cell types comprising different particle sizes, different shapes, different concentrations or the like. This adaptability renders the proposed method exceptionally flexible.

The term "image-related data" is to be understood in a broad sense. It represents at least one image and can in this sense be a normal photographic representation. In addition, the term "image-related data" may comprise monitoring sensor data of other sensors of the monitoring sensor arrangement 17 regarding other properties of the liquid. Such properties may, for example, be flow weight, flow velocity, cell type, carbon-source concentration, nitrogen-source concentration, amino acid concentration, pH, temperature, oxygen concentration, carbon dioxide concentration, conductivity, pressure, DNA concentration, protein concentration or biomass concentration.

In the embodiment according to Fig. 2, here and preferably in the monitoring routine, according to the calculation model, the particle filling level 20 is being calculated based on the different optical properties of the centrifuge chamber content 23-26. These different optical properties are in particular a different translucence, different colour and/or different brightness, of the centrifuge chamber content 23-26 within and outside the particle accumulation. This may for instance be a different colour of the solid particles, preferably cells, caused by characteristics that are specific to a certain particle type, preferably cell type, such as different turbidity, a colour-brightness, and/or density-difference within and outside the particle accumulation. The term "particle accumulation" means here the mass of accumulated particles within a centrifuge chamber 9-12.

In a preferred embodiment, in the monitoring routine, according to the calculation model, the phase boundary 27 between the particle accumulation and the particle free rest of the centrifuge chamber content 23-26 is detected in the optical image 22. Such a phase boundary 27 may develop due to differences in the composition of the particle accumulation and the particle free rest of the centrifuge chamber content 23-26, such as a difference in density, a difference in affinity, e.g. hydrophilic or hydrophobic properties, a difference in colour, translucence and/or brightness. Subsequently, the particle filling level 20 is being calculated from the position of the phase boundary 27 based on the calculation model. The phase boundary 27 may be detected by image processing, for example by an algorithm for line recognition. It may also be detected simply by determining two areas of different optical properties, such as contrast, brightness or the like within the optical image 22 representing the centrifuge chamber content 23-26. These different optical properties preferably represent the particle accumulation on the one hand (e.g. comprising a lower brightness) and the particle free rest (e.g. comprising a higher brightness) of the centrifuge chamber content 23-26 on the other hand.

Preferably, as can be seen in Fig. 1 and Fig. 2 the at least one optical sensor 18 of the monitoring sensor arrangement 17 is a camera unit, preferably a 2D or 3D camera. Exemplary 2D cameras to be used are two-dimensional CCD-array-sensors used for video cameras and digital cameras as well as CMOS-sensors used for smartphones and tablets. The optical sensor 18 is preferably located inside or outside the fluidized bed centrifuge 3, further preferably inside or outside the centrifuge rotor chamber.

The viewing direction of the optical sensor 18, here and preferably the camera unit, is preferably basically parallel to the geometrical centrifuge axis 13. It may, however, be inclined, which may be advantageous in terms of the optimized use of the available space. The term "basically parallel" means here no ideal parallel line in a mathematical sense, but in a colloquial sense, wherein the camera unit is for the better part arranged parallel (see Fig. 2) to the geometrical centrifuge axis 13.

In Fig. 2, two alternatives for the light arrangement 28, 29 are displayed. In the first alternative, the light of the light arrangement 29 is shining through the centrifuge chamber content 23 - 26 and onto the monitoring sensor arrangement 17. This improves the reproducibility of the measurements by the optical sensor 18, independently from any other surrounding conditions. In an alternative embodiment included in Fig. 2 the light of the light arrangement 28 is shining onto the centrifuge chamber content 23-26 and reflected onto the monitoring sensor arrangement 17.

Preferably, the monitoring sensor arrangement 17 and/or the light arrangement 28, 29 is/are being synchronized with the turning of at least one centrifuge chamber 9-12. The term "synchronized" means the bidirectional temporal combination of events to operate a system in unison.

According to an especially preferred embodiment, a stroboscopic LED or an array of LED's and/or the camera unit are synchronized with the turning of the at least one centrifuge chamber 9-12. In particular, the synchronized operation of the monitoring sensor arrangement 17 makes sure to fade out any optical information, which is not related to the centrifuge chamber 9-12 itself.

In the preferred embodiment according to Fig. 2, the particle filling level 20 represents the range between a particle free state of the centrifuge chamber 9-12 and a state of maximum particle accumulation. This state of maximum particle accumulation is defined by the state, which is the borderline to particle breakthrough and which is followed by particle breakthrough when proceeding with the loading cycle.

As also shown in Fig. 2, here and preferably, the fluidized bed centrifuge 3 comprises a monitoring aperture 30 in a monitoring panel, through which the centrifuge chamber contents 23-26 may be monitored. The monitoring aperture is assigned and aligned to the optical sensor 18. It is preferred that the fluidized bed centrifuge 3 comprises exactly one monitoring aperture 30 in a monitoring panel for monitoring the centrifuge chamber contents 23-26 of all the centrifuge chambers 9-12. In this case, the monitoring panel with the monitoring aperture 30 is preferably fixed, while the centrifuge chamber 9-12 is turning around the centrifuge axis 13. Alternatively, according to an especially preferred embodiment, the fluidized bed centrifuge 3 comprises one monitoring aperture 30 in a monitoring panel for each centrifuge chamber 9-12, wherein each monitoring aperture 30 is aligned to the assigned centrifuge chamber 9-12. In this preferred embodiment, each monitoring aperture 30 in the respective monitoring panel is moving with the respective centrifuge chamber 9-12 around the centrifuge axis 13.

Just as a matter of completeness it may be noted that, according to Fig. 1, the bioprocess installation 2 comprises a cultivation setup 31 with at least one upstream unit 32 for producing the bioproduct, in particular a bioreactor. As also indicated by Fig. 1, the bioprocess installation 2 comprises a downstream setup 33 with at least one downstream unit 34. This downstream setup 33 is preferably at least one out of the group of filtration setup, viral inactivation setup, chromatography setup and/or viral filtration setup. The at least one downstream unit 34 is preferably at least one out of the group of microfiltration unit, ultrafiltration unit, capture chromatography unit, viral inactivation unit, diafiltration unit, intermediate (purification) chromatography unit, polishing chromatography unit, viral filtration unit and/or sterile filtration unit.

During the proposed adjusting routine, the at least one adjusted parameter of the clarification setup 1 is the pumping performance, preferably the volumetric flow rate, of at least part of the pumps 5-8 of the pumping arrangement 4, each being assigned to one of the centrifuge chambers 9-12.

The adjusting strategy may be realized in numerous ways, without leaving the scope of the invention. The adjusting strategy is generally directed to controlling the loading of at least part of the centrifuge chambers 9-12. In the following, for a compact presentation of the invention, the preferred variant of controlling all of the centrifuge chambers 9-12 according to the adjusting strategy is described. However, all of those explanations given are equally applicable for the variant of controlling only part of the centrifuge chambers 9-12 according to the adjusting strategy.

Depending on the adjusting strategy, the particle filling levels of the centrifuge chambers 9-12 are being controlled. For the present invention, this is being done based on the particle filling levels that have been derived from the monitoring sensor data 19, preferably from the image-related data 21.

The adjustment strategy may be directed to control the particle filling levels of at least part of the centrifuge chambers 9-12 during the loading cycle to reach a target, preferably a target particle filling level or a target time course of filling level.

In an especially preferred embodiment, this means, that according to the adjusting strategy, a target in the form of a maximum filling level is defined, that each centrifuge chamber 9-12 is controlled to reach. If the target is reached by one of the chambers 9-12, loading is terminated only for this particular centrifuge chamber 9-12, while the other centrifuge chambers 9-12 are continued to be loaded and controlled based on the monitoring sensor data 19.

The adjusting strategy may well be modified such that all centrifuge chambers 9-12 are being filled with particles in a synchronized way, such that the respective target is being reached simultaneously by all centrifuge chambers 9-12.

The above noted target may be an individual target for each centrifuge chamber 9-12, which particle filling level is to be controlled. Alternatively, the target may be a shared target for all centrifuge chambers 9-12, which particle filling levels are to be controlled. Here, the invention displays its full potential in view of flexibility.

As indicated above, the adjusting strategy includes the particle filling levels of at least part of the centrifuge chambers 9-12 being controlled relatively to each other based on the monitoring sensor data 19, preferably the image related data 21. This may include any synchronized control of the particle filling levels, which may be continuous or discontinuous.

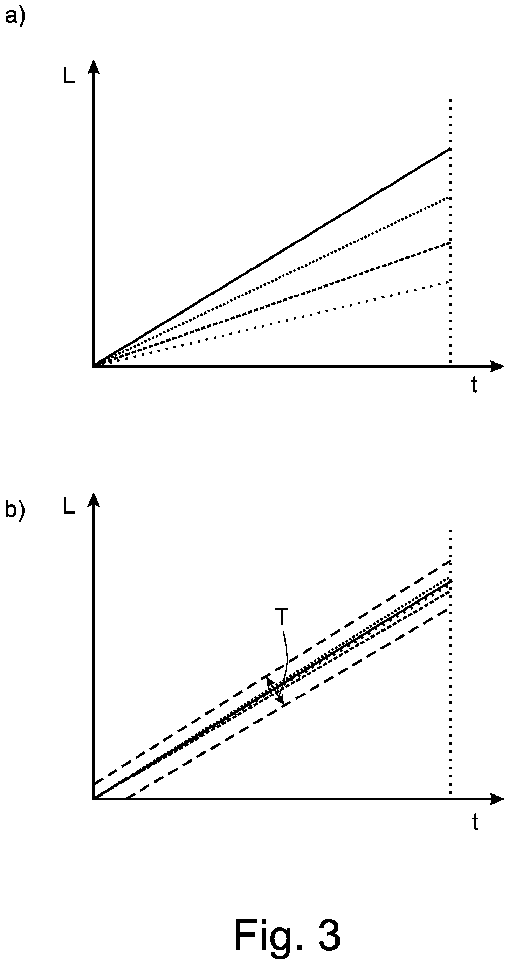

For example, according to a preferred adjusting strategy, the particle filling levels of the centrifuge chambers 9-12 are being controlled to converge based on the monitoring sensor data 19, preferably the image related data 21. In addition or as an alternative, according to the adjusting strategy, during the loading cycle, the particle filling levels of the centrifuge chambers 9-12 are being controlled to deviate from each other or from an average particle filling level by less than a predefined threshold T. This is indicated in Fig. 3b, in which the particle filling level L is displayed for the centrifuge chambers 9-12 during the loading cycle. In this graph, each centrifuge chamber 9-12 is assigned a different line pattern.

As a comparison, Fig. 3a displays the particle filling level L for the centrifuge chambers 9-12 during the loading cycle without applying a proposed adjusting strategy. It becomes clear, that without a proposed adjusting strategy, the filling levels L of the centrifuge chambers 9-12 diverge, such that at the end of the loading cycle a rather random distribution of particle filling levels is to be noted, which compromises not only the efficiency, but also of the reproducibility of the centrifugation result.

The proposed solution may also be advantageous in view of the aspect of balancing the centrifuge 3, in particular the rotor 15, which is carrying the centrifuge chambers 9-12. This is because an uneven filling of the centrifuge chambers 9-12 with particles generally leads to an unbalance with respect to the centrifuge axis 13. As noted above, in an unbalanced state, the inertia axis 35 of the rotor 15 with respect to the centrifuge axis 13 is offset the centrifuge axis 13. This unbalanced state causes vibrations and in the extreme case even mechanical wear or breakage. In any case, the unbalanced state compromises efficiency of the centrifugation, as those vibrations influence the fluid flow and reduces the controllability of the process. Advantageously, a balanced operation may be achieved or at least be supported by the proposed solution.

In detail, according to a preferred adjusting strategy, the volumetric flow rates of the pumps 5-8 assigned to centrifuge chambers 9-12 are controlled for mechanically balancing the fluidized bed centrifuge 3 based on the monitoring sensor data 19, preferably the image related data 21, based on a balancing model. The balancing model preferably represents the interrelation between a difference between particle filling levels of the centrifuge chambers 9-12 and an unbalance of the fluidized bed centrifuge 3.

According to an especially preferred embodiment, the balancing model can be adapted to different bioprocesses comprising different properties, preferably by the electronic process control 14 and/or the user. Different bioprocesses preferably employ different cell broth compositions, consisting of different media and/or cell types.

Although different media invariably contain at least a carbon source, a nitrogen source, water, salts, and micronutrients, a variety of media exist and they might differ with regards to liquid properties, such as liquid density, viscosity or the like. Preferred cell types used for a bioprocess are bacterial, plant or mammalian cells including stem cells or immune cells such as CAR-T cells for the treatment of cancer, as mentioned earlier. The cells of choice used for the bioprocess grow in specially designed media, which supply the nutrients required by the respective organisms or cells. However, such different cell types are also accompanied by differences in cell densities, different cell viabilities, different cell morphologies, different cell diameters, or the like, which are taken into account by the balancing model.

Preferably, and to be understood just as an example, the balancing model is adaptable for mechanically balancing the fluidized bed centrifuge 3 in a bioprocess employing a cell type comprising a higher density. Since particles of higher density exhibit a larger mass per volume in comparison to particles of lower density, a larger mass per volume is loaded into the centrifuge chambers 9-12, when comparing to a bioprocess employing a cell type of lower density. At the same cell concentration and volumetric flow rate, the centrifuge chambers 9-12 would hence exhibit a higher mass at a given particle filling level, when loaded with a cell type comprising a higher density. In this case, and according to the adjusting strategy, the volumetric flow rate of at least one pump 5-8 assigned to the respective centrifuge chamber 9-12 is adapted, preferably, at least the pumps assigned to opposing centrifuge chambers 9-12 are adapted relatively to each other, for mechanically balancing the fluidized bed centrifuge 3, based on the adapted balancing model. This adaptability of the balancing model allows the supply of the cell broth, in particular of different cell broths, to different centrifuge chambers 9-12, which renders the present invention particularly flexible.

In another embodiment, the clarification setup 1 comprises a vibration sensor for detecting the presence and/or the amplitudes of vibrations within the fluidized bed centrifuge 3, wherein according to the adjusting strategy, the volumetric flow rates of at least part of the pumps 5-8 assigned to the centrifuge chambers 9-12 are controlled for minimizing the amplitudes of vibrations within the fluidized bed centrifuge 3 detected by the vibration sensor. This adjusting strategy may well be applied, if no balancing model is available. It may, however, be applied in addition to the control based on a balancing model. In any case, reducing the vibration is preferably achieved in an iterative approach, changing the pumping performance, preferably the volumetric flow rate, of the pumps 5-8 one after the other and adapting the pumping performance accordingly. With this it is even possible to counteract an imbalance of the rotor itself or its mechanical support, just by accordingly filling the centrifuge chambers 9-12 with particles.

## Claims

1. Method for operating a clarification setup (1) of a bioprocess installation (2), wherein the clarification setup (1) comprises a fluidized bed centrifuge (3) for the clarification of a cell broth by centrifugation and a pumping arrangement (4) with at least two pumps (5-8) assigned to the fluidized bed centrifuge (3), wherein the fluidized bed centrifuge (3) comprises at least two centrifuge chambers (9-12), which are being turned around a single geometrical centrifuge axis (13), wherein the bioprocess installation (2) comprises an electronic process control (14) for controlling at least the fluidized bed centrifuge (3) and the pumping arrangement (4), wherein the fluidized bed centrifuge (3) is being operated in a forward operation for a particle loading cycle and/or a particle washing cycle and in a backward operation for a particle discharging cycle, wherein during the particle loading cycle, cell broth loaded into the centrifuge chamber (9-12) proceeds to form a growing particle accumulation in the centrifuge chamber (9-12), wherein the clarification setup (1) comprises a monitoring sensor arrangement (17) with at least one, preferably optical, sensor (18) for producing monitoring sensor data, which are being transmitted to the electronic process control (14),
**characterized in**
**that** in a monitoring routine, for at least part of the centrifuge chambers (9-12), monitoring sensor data (19) are being produced individually by the monitoring sensor arrangement (17) and that during the loading cycle, in an adjusting routine, at least one parameter of the clarification setup (1) is being adjusted by the electronic process control (14) based on the monitoring sensor data (19) as to control the loading of the cell broth for at least part of the centrifuge chambers (9-12) individually according to a predefined adjusting strategy.

2. Method according to claim 1, **characterized in that** in the monitoring routine, image-related data (21) representing an optical image (22) of the centrifuge chamber content (23-26) are being produced as the monitoring sensor data (19) by the monitoring sensor arrangement (17).

3. Method according to any one of the preceding claims, **characterized in that** the monitoring sensor data (19) represent the particle filling level (20) of the respective centrifuge chambers (9-12), and/or, that the particle filling level (20) is being calculated by the electronic process control (14) from the monitoring sensor data (19) based on a calculation model.

4. Method according to any one of the preceding claims, **characterized in that** the at least one adjusted parameter of the clarification setup (1) is the pumping performance, preferably the volumetric flow rate, of at least part of the pumps (5-8) of the pumping arrangement (4), each being assigned to one of the centrifuge chambers (9-12).

5. Method according to any one of the preceding claims, **characterized in that** during the loading cycle, the particle filling levels of at least part of the centrifuge chambers (9-12) are being controlled according to the adjusting strategy.

6. Method according to any one of the preceding claims, **characterized in that** according to the adjusting strategy, during the loading cycle, the particle filling levels of at least part of the centrifuge chambers (9-12) are being controlled to reach a target, preferably a target particle filling level or a target time course of filling level.

7. Method according to any one of the preceding claims, **characterized in that** the target is an individual target for each centrifuge chamber (9-12), which particle filling level is to be controlled, or, that the target is a shared target for all centrifuge chambers (9-12), which particle filling levels are to be controlled.

8. Method according to any one of the preceding claims, **characterized in that** according to the adjusting strategy, the particle filling levels of at least part of the centrifuge chambers (9-12) are being controlled relatively to each other.

9. Method according to any one of the preceding claims, that according to the adjusting strategy, the particle filling levels of at least part of the centrifuge chambers (9-12) are being controlled to converge based on the monitoring sensor data (19), preferably the image related data (21).

10. Method according to any one of the preceding claims, **characterized in that** according to the adjusting strategy, the particle filling levels of at least two centrifuge chambers (9-12) are being controlled based on the monitoring sensor data (19), preferably the image related data (21), to deviate from each other or from an average particle filling level by less than a predefined threshold.

11. Method according to any one of the preceding claims, **characterized in that** according to the adjusting strategy, the volumetric flow rates of at least two pumps (5-8) assigned to centrifuge chambers (9-12) are controlled for mechanically balancing the fluidized bed centrifuge (3) based on the monitoring sensor data (19), preferably the image related data (21), based on a balancing model, preferably, that the balancing model represents the interrelation between a difference between particle filling levels of at least two centrifuge chambers (9-12) and an unbalance of the fluidized bed centrifuge (3).

12. Method according to any one of the preceding claims, **characterized in that** the clarification setup (1) comprises a vibration sensor for detecting the presence and/or the amplitudes of vibrations within the fluidized bed centrifuge (3) and that according to the adjusting strategy, the volumetric flow rates of at least part of the pumps (5-8) assigned to the centrifuge chambers (9-12) are controlled for minimizing the amplitudes of vibrations within the fluidized bed centrifuge (3) detected by the vibration sensor.

13. Clarification setup (1) of a bioprocess installation (2) for performing the method according to any one of the preceding claims, wherein the clarification setup (1) comprises a fluidized bed centrifuge (3) for the clarification of a cell broth by centrifugation and a pumping arrangement (4) with at least one pump (5-8) assigned to the fluidized bed centrifuge (3), wherein the fluidized bed centrifuge (3) comprises at least two centrifuge chambers (9-12), each of which is being turned around a single geometrical centrifuge axis (13), wherein the bioprocess installation (2) comprises an electronic process control (14) for controlling at least the fluidized bed centrifuge (3) and the pumping arrangement (4), wherein the fluidized bed centrifuge (3) is being operated in a forward operation for a particle loading cycle and/or a particle washing cycle and in a backward operation for a particle discharging cycle, wherein the particle loading cycle, cell broth loaded into the centrifuge chamber (9-12) proceeds to form a growing particle accumulation in the centrifuge chamber (9-12), wherein the clarification setup (1) comprises a monitoring sensor arrangement (17) with at least one, preferably optical, sensor (18) for producing monitoring sensor data, which are being transmitted to the electronic process control (14),
**characterized in**
**that** in a monitoring routine, for at least part of the centrifuge chambers (9-12), monitoring sensor data (19) are being produced individually by the monitoring sensor arrangement (17) and that during the loading cycle, in an adjusting routine, at least one parameter of the clarification setup (1) is being adjusted by the electronic process control (14) based on the monitoring sensor data (19) as to control the loading of the cell broth for at least part of the centrifuge chambers (9-12) individually according to a predefined adjusting strategy.

14. Electronic process control of the clarification setup according to claim 13, **characterized in that** the electronic process control is designed for performing the method according to any one of the claims 1 to 12.
